# EUROPEAN PATENT APPLICATION

(11) **EP 1 104 667 A1**
(43) Date of publication of application: **06.06.2001**
(21) Application number: 99123656.3
(22) Date of filing: 29.11.1999
(51) Int. Cl.: A61F 13/15

(54) **Disposable absorbant article having fecal management layer**

(71) Applicant: THE PROCTER & GAMBLE COMPANY, Cincinnati, Ohio 45202 (US)
(72) Inventor: Divo, Michael, 61381 Friedrichsdorf (DE); Lankhof, John Peter, 61352 Bad Homburg (DE)
(74) Representative: Mather, Peter Geoffrey

(57) **Abstract**

The present invention provides disposable absorbent articles which combine the ability to acquire and store fecal material with an improved wearing comfort.

## Description

### FIELD OF THE INVENTION

The present invention relates to absorbent articles for retaining body fluids such as urine, menses, or fecal material, and in particular to their ability to acquire and retain aqueous based materials. The invention further relates to disposable absorbent articles such as baby diapers or training pants, adult incontinence products, and feminine hygiene products.

### BACKGROUND

It has been a trend in recent absorbent articles to provide the capability to handle high viscosity liquids such as faeces. Because of the high viscosity of such liquids, it is desirable that the respective acquisition structures provide large open pores in order to readily accept such liquids. For example such structures are described in WO 95/05139 (Roe), in PCT/US97/20840 (Bast et al.), and in PCT/US98/24389 (Roe et al.).

Large open pores, however, may lead to reduced contact area with the skin of the wearer. Accordingly, the local pressure throughout this small contact areas between absorbent article and skin is comparatively high. Such high pressures may in turn lead to reduced wearing comfort through, for example, increased skin imprinting.

Therefore, it is an object of the present invention to provide an absorbent article which overcomes the problems posed by the prior art absorbent articles.

### SUMMARY OF THE INVENTION

The present invention provides an absorbent article which combines the ability to acquire and store fecal material with an improved wearing comfort, in particular with reduced skin imprinting leading to a reduced level of pressure marks. The absorbent article of the present invention comprises a liquid pervious structured carrier, a liquid impervious backsheet at least partially peripherally joined to said structured carrier, a liquid storage member positioned intermediate said structured carrier and said backsheet, and a liquid handling member positioned intermediate said structured carrier and said liquid storage member.

### BRIEF DESCRIPTION OF THE FIGURES

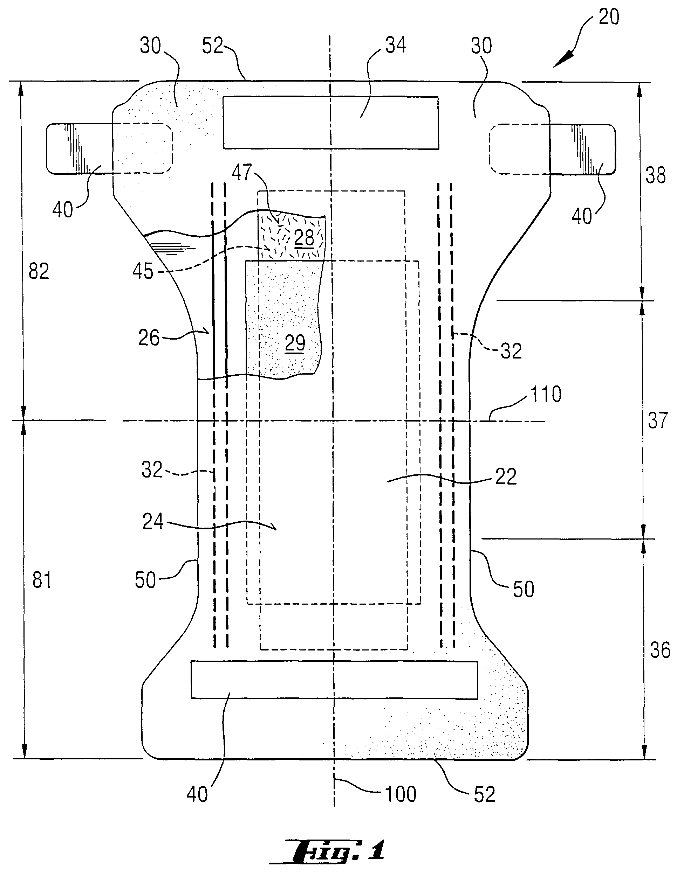

Figure 1 is a plan view of an absorbent article embodiment of the present invention having portions cut away to reveal the underlying structure, the garment-facing surface of the diaper facing the viewer.

### DETAILED DESCRIPTION OF THE INVENTION

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a co-ordinated entity so that they do not require separate manipulative parts like a separate holder and liner. A preferred embodiment of an absorbent article of the present invention is the unitary disposable absorbent article, diaper 20, shown in Figure 1. As used herein, the term "diaper" refers to an absorbent article generally worn by infants and adult incontinent persons and is worn about the lower torso of the wearer. The present invention is also applicable to other absorbent articles such as incontinence briefs, incontinence undergarments, absorbent inserts, diapers holders and liners, feminine hygiene garments, and the like.

Figure 1 is a plan view of the diaper 20 of the present invention in a flat-out, state with portions of the member being cut-away to more clearly show the construction of the diaper 20. The portion of the diaper 20 which faces the wearer is oriented towards the viewer. As shown in Figure 1, the diaper 20 preferably comprises a liquid pervious structured carrier 24; a liquid impervious backsheet 26; an liquid storage member 28, which is preferably positioned between at least a portion of the structured carrier 24 and the backsheet 26; a liquid handling member 29 positioned intermediate the structured carrier 24 and the liquid storage member 28; side panels 30; elasticized leg cuffs 32; an elastic waist feature 34; and a fastening system generally designated 40.

Structured carriers which are suitable for the present invention include apertured nonwoven web materials having an aperture size of about 2-4 mm² and have an open area of about 15-30%. A suitable structured carrier is commercially available under the designation "VP 13-96-378nc" from Christian Heinrich Sandler GmbH & Co. KG of Schwarzenbach/Saale, Germany. Another suitable structured carrier may be manufactured by selectively aperturing a one-ply or a two-ply nonwoven web material. A suitable process for selectively aperturing nonwoven web materials is disclosed in WO-A-97/11662 (Benson et al.). During such aperturing the nonwoven material may be stretched to reduce the basis weight to about half of the original basis weight. All process settings should be chosen by the skilled practitioner to obtain an apertured nonwoven web material satisfying the above aperture size and open area requirements. Suitable one-ply web materials include spunbond nonwoven web materials comprising 50/50 PE/PP bico fibers and/or 80/20 PE/PP bico fibers and having a basis weight of about 60 grams per square meter and further include nonwoven web materials comprising PE fibers having a basis weight of 60 grams per square meter. Suitable one-ply nonwoven web materials are available from BBA Nonwovens Washougal Inc. of Washougal, Washington, USA, under the designation "13B (29307032)" as well as from Corovin GmbH of Peine, Germany, under the designation "Corolind PE 60 gsm". Suitable two-ply nonwoven web materials include materials comprising a body facing nonwoven layer comprising 50/50 PE/PP or 80/20 PE/PP bico fiber with a basis weight of about 30 grams per square meter and an inner nonwoven layer comprising 50/50 PE/PP bico fibers with a basis weight of about 30 grams per square meter. Suitable two-ply nonwoven web materials may comprise body facing layers available from BBA Nonwovens of Simpsonville, South Carolina, USA, under the designations "17848-14", "17848-12", "17848-11", "178848-0" or from BBA Nonwovens Washougal Inc. of Washougal, Washington, USA, under the designations "13C (29307036)" and "13J (29306015)". Suitable two-ply nonwoven web materials may comprise garment facing layers available from BBA Nonwovens of Simpsonville, South Carolina, USA, under the designations "17848-14", "17848-12", "17848-11" or from BBA Nonwovens Washougal Inc. of Washougal, Washington, USA, under the designations "13A (29307027)" and "13H (29306008)". An anti-static lubricant mixture suitable for making fibers cardable may be added to at least one surface of the structured carrier of the present invention at a level of about 0.5-1% by weight and is commercially available from Schill & Seilacher GmbH & Co. Chem. Fabriken of Böblingen, Germany, under the designation "Silastol M12". Other suitable apertured structured carriers include selectively apertured carded nonwoven web materials with a basis weight of about 30 grams per square meter comprising PP fibers and optionally 50/50 PE/PP copolymer fibers for example available from Christian Heinrich Sandler GmbH & Co. KG of Schwarzenbach/Saale, Germany, under the designations "SAN4112" and "SAN393".

The body facing surface of the structured carrier may comprise an effective amount of a skin care composition which is semi-solid or solid at 20° C and which is partially transferable to the wearer's skin. In preferred embodiment of the absorbent article of the present invention, the absorbent article additionally comprises an skin care composition which is at least partially transferable to the skin of the user during the intended use. Preferably, such an oil-containing composition is positioned on a user facing surface of the absorbent article. The oil-containing composition may also be deployed in such a way that it is only released at the time of intended use such as being microencapsulated. Skin care compositions suitable for the absorbent article of the present invention are described for example in WO96/16682 (Roe et al.).

Liquid handling members which are suitable for the present invention by combining efficacy in handling fecal material with high wearing comfort include members comprising corrugated hydrophilic nonwoven layers (basis weight about 40 grams per m² to about 60 grams per m²) comprising polyester fibers between 3 denier and 6 denier. A suitable process for manufacturing the liquid handling member of the present invention is disclosed in WO-A-99/25293 (Bast et al.). Suitable liquid handling member are available from Minnesota Mining and Manufacturing Company of Minneapolis, Minnesota, USA, under the designations "XPC-99-131", "XPC-99-161", "XPC-99-194", "XPC-99-240", and "XPC-99-243G", and "XPC-99-246". The liquid handling member of the present invention may be shaped to better adapt to the anatomy of the wearer in particular by having a reduced transverse dimension in the crotch region.

Adhesives suitable for joining the structured carrier to the liquid handling member are available from H. B. Fuller GmbH of Lüneburg, Germany, under the designations "D875BD15", "D3150", and "3151", as well as from National Starch & Chemical Limited of Slough, United Kingdom, under the designations "034-5646" and "521A" as well as from AtoFindley Nederland B.V., AD Roosendaal, The Netherlands, under the designations "HX9238-03", "HX9310-01", "HX9308-01", "HX7131-01", and "HX7127-01".

The liquid storage member 28 may be any absorbent means which is generally compressible, conformable, non-irritating to the wearer's skin, and capable of absorbing and retaining liquids such as urine and other certain body exudates. As shown in Figure 1, the liquid storage member 28 has a garment surface, a body surface, side edges, and waist edges. The liquid storage member 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the liquid storage member 28 may also be varied (e.g., the liquid storage member 28 may have varying caliper zones, a hydrophilicity gradient, a pore size gradient, a superabsorbent gradient, or lower average density and lower average basis weight acquisition zones; or may comprise one or more layers or structures). The total absorbent capacity of the liquid storage member 28 should, however, be compatible with the design loading and the intended use of the diaper 20. Further, the size and absorbent capacity of the liquid storage member 28 may be varied to accommodate wearers ranging from infants through adults. The liquid storage member of the present invention may be shaped to better adapt to the anatomy of the wearer in particular by having a reduced transverse dimension in the crotch region.

Exemplary absorbent structures for use as the liquid storage member 28 are described in US Patent 4,610,678 entitled "High-Density Absorbent Structures" issued to Weisman et al. on September 9, 1986; US Patent 4,673,402 entitled "Absorbent Articles With Dual-Layered Cores" issued to Weisman et al. on June 16, 1987; US Patent 4,888,231 entitled "Absorbent Core Having A Dusting Layer" issued to Angstadt on December 19, 1989; and US Patent 4,834,735, entitled "High Density Absorbent Members Having Lower Density and Lower Basis Weight Acquisition Zones", issued to Alemany et al. on May 30, 1989. Suitable for materials for the acquisition zones include stiffened cellulosic fibers available from Weyerhaeuser of Tacoma, Washington, USA, under the designations "CMC517", "CMF Trial 104", and "CMF Trial 111".

The backsheet 26 is positioned adjacent the garment surface of the liquid storage member 28 and is preferably joined thereto by attachment means (not shown) such as those well known in the art. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

For example, the backsheet 26 may be secured to the liquid storage member 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in US Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in US Patent 3,911,173 issued to Sprague, Jr. on October 7, 1975; US Patent 4,785,996 issued to Ziecker, et al. on November 22, 1978; and U.S. Patent 4,842,666 issued to Werenicz on June 27, 1989. Each of these patents is incorporated herein by reference. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body.

The backsheet 26 prevents the exudates absorbed and contained in the liquid storage member 28 from wetting articles which contact the diaper 20 such as bedsheets and undergarments. The backsheet 26 may thus comprise a woven or nonwoven material, polymeric films such as thermoplastic films of polyethylene or polypropylene, or composite materials such as a film-coated nonwoven material. Preferably, the backsheet 26 is a thermoplastic film having a thickness of about 0.012 mm (0.5 mil) to about 0.051 mm (2.0 mils). Particularly preferred materials for the backsheet 26 include RR8220 blown films and RR5475 cast films as manufactured by Tredegar Industries, Inc. of Terre Haute, Indiana. The backsheet 26 is preferably embossed and/or matte finished to provide a more cloth-like appearance. Further, the backsheet 26 may permit vapors to escape from the liquid storage member 28 (i.e., be breathable) while still preventing exudates from passing through the backsheet 26.

## Claims

1. An absorbent article comprising a liquid pervious structured carrier, a liquid impervious backsheet at least partially peripherally joined to said structured carrier, a liquid storage member positioned intermediate said structured carrier and said backsheet, and a liquid handling member positioned intermediate said structured carrier and said liquid storage member
characterized in that
said absorbent article combines the ability to acquire and store fecal material with an improved wearing comfort.
